# EUROPÄISCHE PATENTSCHRIFT

(11) **EP 0 584 525 B1**
(45) Veröffentlichungstag und Bekanntmachung des Hinweises auf die Patenterteilung: **07.05.1997**
(21) Anmeldenummer: 93111547.1
(22) Anmeldetag: 19.07.1993
(51) Int. Cl.: A61N 1/05

(54) **Elektrodenvorrichtung**
Electrode device
Dispositif d'électrode

(30) Priorität: 28.08.1992 SE 9202480
(43) Veröffentlichungstag der Anmeldung: 02.03.1994
(73) Patentinhaber: Pacesetter AB, 171 95 Solna (SE)
(72) Erfinder: Nyman, Per, S-182 64 Djursholm (SE); Lindegren, Ulf, S-122 35 Enskede (SE)
(74) Vertreter: Lettström, Richard Wilhelm

(56) Entgegenhaltungen:
- US-A- 4 407 303
- US-A- 4 550 737

## Beschreibung

Die Erfindung betrifft eine Elektrodenvorrichtung zur intrakorporalen Stimulation von Körpergeweben, insbesondere zur intrakardialen Stimulation von Herzgeweben mit einem Elektrodenkabel, das mindestens einen langgestreckten, flexiblen Leiter umfasst und mit einem Elektrodenkopf, der am distalen Ende des Elektrodenkabels angebracht ist, wobei das Elektrodenkabel mit einer Isolierschicht versehen ist.

Es ist von grosser Bedeutung, dass das Elektrodenkabel bei einer derartigen Elektrodenvorrichtung ausreichend biegsam ist, damit es bei einer Einführung in das Herz eines Patienten über eine Vene dieser folgen kann, ohne die Venenwände zu beschädigen. Die Einführung des Elektrodenkabels erfolgt mit Hilfe eines Mandrins, der in den Kanal des Elektrodenkabels eingeführt wird. Der Mandrin besteht aus einem Material, das ausreichend steif ist, damit das Elektrodenkabel in einer Vene verschoben werden kann. In schwierigen Passagen, in denen das Elektrodenkabel stark gebogen werden muss, wird der Mandrin häufig etwas nach hinten verschoben, so dass das distale Ende des Elektrodenkabels maximal biegsam wird. Nachdem das vordere Ende des Elektrodenkabels eine solche Stelle passiert hat, wird der Mandrin wieder an das distale Ende vorgeschoben, damit dieses Ende zum Vorhof oder zur Kammer des Herzens transportiert werden kann, bis der Elektrodenkopf gegen die Herzwand zur Stimulation anliegt. Die Einführung eines Elektrodenkabels in eine Vene mit Hilfe eines Mandrins, der dazu beiträgt, dass das Elektrodenkabel verhältnismässig steif wird, ist daher für den Arzt nicht ganz einfach durchzuführen. Elektrodenvorrichtungen der eingangs genannten Art, bei denen die Elektrodenkabel mittels eines Mandrins durch eine Vene transportiert werden, sind u.a. in den US-PS 4 136 703, 4 402 328 und 4 677 990 beschrieben.

In US-A-4 550 737 wird in Spalte 7, oben vorgeschlagen, daß ein kurzes distales Gewinde einen Herzkatheter mittels Rotation vorwärts bewegen kann.

Der Erfindung liegt die Aufgabe zugrunde, eine Elektrodenvorrichtung der eingangs genannten Art zu schaffen, bei der das Elektrodenkabel mit beibehaltener Geschmeidigkeit und Elastizität mit verhältnismässig einfachen Mitteln und ohne Hilfe eines Mandrins in einer Vene transportiert werden kann.

Diese Aufgabe ist erfindungsgemäss dadurch gelöst, dass an der Aussenwand der Isolierschicht Mitteln angeordnet sind, die sich wendelförmig entlang der Länge des Elektrodenkabels erstrecken. Durch Rotieren des Elektrodenkabels um seine Längsachse wird das Elektrodenkabel mit Hilfe dieser Mittel in einer Vene vorwärts geschraubt. Indem das Elektrodenkabel nicht mit einem Kanal für ein Mandrin versehen sein muss, kann der Durchmesser des Kabels klein gehalten werden, und indem in Verbindung mit einer Implantation kein Mandrin erforderlich ist, kann das Elektrodenkabel seine Geschmeidigkeit beibehalten.

In einer Weiterbildung der Erfindung wird vorgeschlagen, dass die Mittel mindestens ein von der Isolierschicht herausragendes flossenähnliches Teil sind. Die Höhe des flossenähnlichen Teils bzw. der Flosse entlang des Elektrodenkabels kann variieren, so dass die Flosse im Bereich des distalen Endes am Elektrodenkabel höher ist als am übrigen Elektrodenkabel. Die Flosse kann auch in eine Anzahl mit Abstand voneinander an der Isolierschicht angebrachten, längeren oder kürzeren Flossen aufgeteilt sein, die gemeinsam eine Wendelform bilden. Ein derartiger Aufbau kann vorteilhaft sein, wenn das Elektrodenkabel an einer Strecke biegsamer sein soll als der Rest des Kabels. Die Flossen können ferner derart ausgebildet sein, dass diese in einer Parklage gegen die Isolierschicht anliegen und sich entfalten, wenn das Blut in der Vene die Flossen aufgreift.

In einer vorteilhaften Ausbildung der Erfindung wird vorgeschlagen, dass die Mittel mindestens eine Nut in der Isolierschicht sind. Hierdurch kann das Elektrodenkabel extrem schmal gemacht werden. Das Profil der Nut entscheidet, wie effektiv das Elektrodenkabel in einer Vene vorwärts geschraubt werden kann.

In einer Weiterentwicklung der Erfindung wird vorgeschlagen, dass die Mittel teils mindestens ein von der Isolierschicht herausragendes flossenähnliches Teil und teils mindestens eine Nut in der Isolierschicht sind, wobei das Teil und die Nut parallel verlaufen. Durch diesen Aufbau wird eine effektive Vorwärtsverschiebung bei einer Rotation des Elektrodenkabels erhalten.

Eine günstige Ausgestaltung der Erfindung ergibt sich, wenn die Steigung der wendelförmigen Mittel am distalen Ende des Elektrodenkabels geringer ist als an dem übrigen Elektrodenkabel. Durch diese Ausführungsform wird ein effektives Vorwärtsschrauben des Elektrodenkabels erreicht. Ausserdem gibt diese Ausführungsform eine gute Möglichkeit, den Elektrodenkopf an der Herzwand zu verankern, indem das wendelförmige Mittel in das Trabekelwerk einschraubbar ist.

Gemäss der Erfindung wird auch eine Anordnung zur Durchführung einer Rotation der Elektrodenvorrichtung erhalten, bei der die Anordnung ein Teil zum Einräumen des Elektrodenkabels und einen motorgetriebenen Rotor umfasst, dessen Welle parallel zur Längsachse des Elektrodenkabels angeordnet ist, wobei der Rotor mit Mitteln versehen ist, die bei einer Rotation die Mittel des Elektrodenkabels betätigen. Da die Anordnung nicht am Elektrodenkabel befestigt ist, ist es für den Arzt nach einer durchgeführten Implantation des Elektrodenkabels einfach, die Anordnung vom Kabel zu entfernen.

Die Mittel des Rotors, die die Mittel des Elektrodenkabels betätigen, können nach aussen gerichtete Borsten sein, die ausreichend steif sind, um das Elektrodenkabel in Rotation zu bringen, gleichzeitig damit, dass die Borsten das Kabel nicht beschädigen dürfen.

Die Erfindung ist nachfolgend anhand von mehreren, in den Zeichnungen dargestellten Ausführungsbeispielen näher erläutert. Es zeigen:
- FIG 1 bis 4: perspektivische Ansichten von Elektrodenvorrichtungen nach der Erfindung in verschiedenen Ausführungensformen,
- FIG 5: einen Querschnitt durch die in der FIG 4 dargestellten Elektrodenvorrichtung nach der Schnittlinie V-V und
- FIG 6: eine Seitenansicht einer Anordnung zur Durchführung einer Rotation von einer der Elektrodenvorrichtungen nach den FIG 1 bis 4.

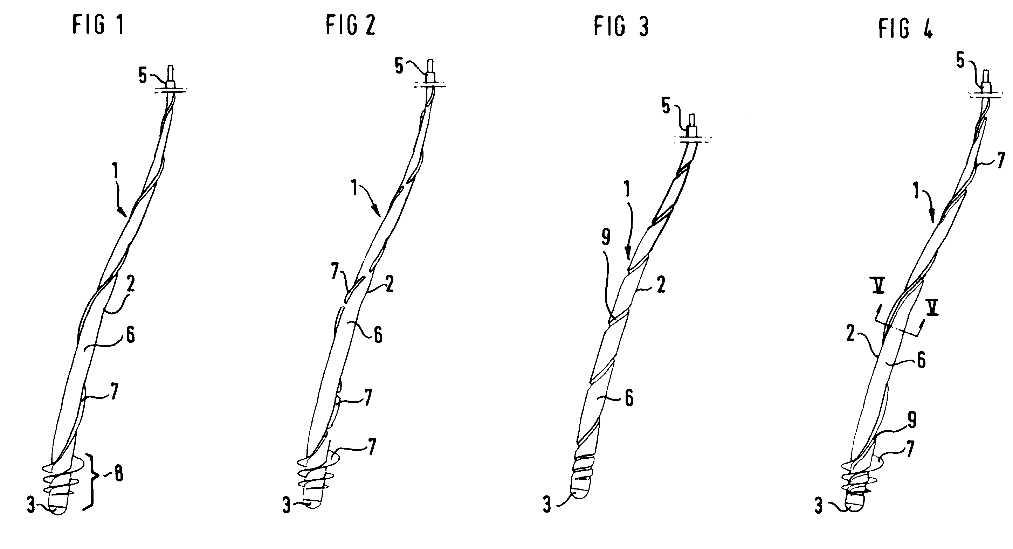

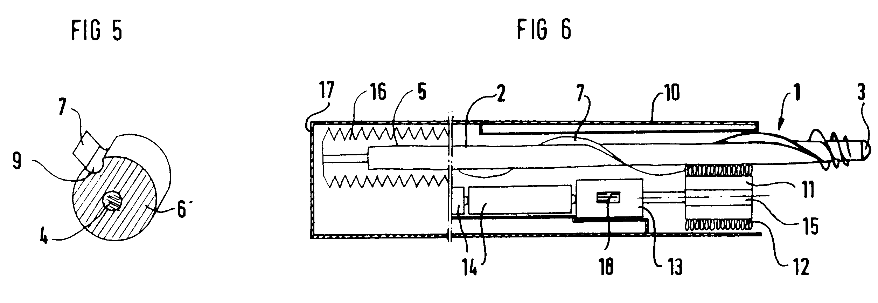

In der FIG 1 ist eine Elektrodenvorrichtung 1 zur intrakardialen Stimulation eines Herzens abgebildet. Die Elektrodenvorrichtung 1 besteht aus einem Elektrodenkabel 2, an dessen distalen Ende ein Elektrodenkopf 3 zur Stimulation von Herzgeweben eines Patienten angebracht ist. Das Elektrodenkabel 2 umfasst einen flexiblen, erst in der FIG 5 gezeigten Leiter 4, der sich vom proximalen Ende 5 des Elektrodenkabels 2 bis zum Elektrodenkopf 3 erstreckt. Das Elektrodenkabel 2 ist ferner mit einer Isolierschicht 6 für den Leiter 4 versehen. Die Aussenwand der Isolierschicht 6 ist mit einem herausragenden flossenähnlichen Teil 7 versehen, das sich wendelförmig entlang der Länge des Elektrodenkabels 2 erstreckt. Die Steigung der wendelförmigen Flosse 7 am distalen Ende des Elektrodenkabels ist in diesem Ausführungsbeispiel geringer als die Steigung am übrigen Elektrodenkabel 2. Bei einer Implantation der Elektrodenvorrichtung 1 in einen Patient erfolgt eine Rotation des Elektrodenkabels 2 z.B. mit Hilfe einer später in der Anmeldung beschriebenen Anordnung. Diese Anordnung ist auch mit Mitteln versehen, die das Elektrodenkabel 2 in einer Vene vorwärts verschieben können. Das Elektrodenkabel 2 wird nun mittels der Flosse 7 vorwärts geschraubt, bis das Kabel 2 eine gewünschte Endposition erreicht hat. Wenn der Elektrodenkopf 3 in der Kammer gegen die Herzwand des Patienten appliziert werden soll, dient die Flosse 7 auch als Verankerungsmittel, da das distale Ende 8 des Elektrodenkabels 2 in das Trabekelwerk eingeschraubt werden kann.

In der FIG 2 ist gezeigt, dass die Flosse 7 in eine Anzahl mit Abstand voneinander angebrachten längeren oder kürzeren Flossen 7 aufgeteilt sein kann, die gemeinsam eine Wendelform entlang der Länge des Elektrodenkabels 2 bilden. Eine solche Ausführung kann vorteilhaft sein, wenn das Elektrodenkabel noch geschmiediger sein soll als ein Elektrodenkabel mit einer durchgehenden, nicht unterbrochenen Flosse.

In der FIG 3 ist dargestellt, dass die Isolierschicht 6 des Elektrodenkabels statt mit einer Flosse mit einer Nut 9 versehen ist, die sich wie die Flosse 7 wendelförmig entlang der Länge des Elektrodenkabels 2 erstreckt. In diesem Ausführungsbeispiel ist die Steigung der Nut 9 geringer als die in der FIG 1 und 2 gezeigte Steigung der Flosse 7.

In der FIG 4 ist dargestellt, dass die Flosse 7 mit einer in der Isolierschicht vorgesehenen Nut 9 kombiniert werden kann, die wendelförmig und parallel zueinander entlang des Elektrodenkabels 2 verlaufen. Die FIG 5, die ein Querschnitt durch das Elektrodenkabel 2 nach FIG 4 ist, zeigt eine Vergrösserung einer solchen Ausführungsform des Elektrodenkabels 2. Durch diese Ausführungsform kann das Elektrodenkabel bei einer Rotation schneller in das Medium transportiert werden. In dieser FIG 5 wird auch gezeigt, dass das Elektrodenkabel 2 verhältnismässig dünn gemacht werden kann, da ein Kanal für einen Mandrin nicht erforderlich ist.

FIG 6 zeigt ein Beispiel einer Anordnung zur Durchführung einer Rotation der Elektrodenvorrichtung. Die Anordnung kann eine Verpackung 10 oder ein Teil einer Verpackung 10 für die Elektrodenvorrichtung 1 sein. In der Verpackung 10 ist ein Rotor 11 angeordnet, der mit nach aussen gerichteten Borsten 12 versehen ist, wobei der Rotor 11 mittels eines Motors 13 angetrieben wird. Der Motor 13, der mit einer Reguliervorrichtung 18 für die Geschwindigkeit versehen ist, kann vorzugsweise mittels Batterien 14 angetrieben werden. Die Welle 15 des Rotors 11 ist parallel zur Längsachse des verpackten Elektrodenkabels 2 angebracht. Wenn der Motor 12 nun den Rotor 11 derart antreibt, daß er um dessen eigene Welle 15 rotiert, bringen die gegen die Isolierung und die Flossen 7 angeordneten Borsten 12 das Elektrodenkabel 2 um seine eigene Längsachse zu rotieren. Das proximale Ende 5 des Elektrodenkabels 2 ist ausserdem in einem in der Verpackung 10 vorhandenen ausgezogenen balgförmigen Teil 16 angebracht, das an einer Wand 17 der Verpackung 10 lösbar befestigt ist. Bei einer Implantation kann das Teil 16 von der Wand 17 gelöst werden, wobei es sich zusammenzieht und dabei das rotierende Kabel 2 vorwärts schiebt, so dass es wie bereits beschrieben, sich in die Vene hineinschraubt.

Die beschriebene Anordnung braucht nicht notwendigerweise eine Verpackung oder ein Teil einer Verpackung für die Elektrodenvorrichtung 1 zu sein, sondern kann auch ein getrenntes Teil sein, in das die Elektrodenvorrichtung vor einer Implantation plaziert wird. Das balgförmige Teil 16 kann auch durch andere Verschiebmittel ersetzt werden.

Die beschriebenen Flossen 7 an den Elektrodenkabeln 2 in den FIG 1, 2 und 4 können selbstverständlich nach Bedarf in Bezug auf die Höhe, Breite, Länge und Steigung auch auf ein und demselben Elektrodenkabel variiert werden. Auch die Nut 9 kann nach Bedarf und Wunsch breiter und tiefer gemacht werden als in den in Verbindung mit den FIG 3, 4 und 5 gezeigten Ausführungsbeispielen ist.

## Patentansprüche

1. Elektrodenvorrichtung zur intrakorporalen Stimulation von Körpergeweben,insbesondere zur intrakardialen Stimulation von Herzgeweben mit einem Elektrodenkabel, das mindestens einen langgestreckten, flexiblen Leiter umfasst und mit einem Elektrodenkopf, der am distalen Ende des Elektrodenkabels angebracht ist, wobei das Elektrodenkabel mit einer Isolierschicht versehen ist, **dadurch gekennzeichnet**, dass an der Aussenwand der Isolierschicht (6) Mittel (7, 9) angeordnet sind, die sich wendelförmig entlang der gesamten Länge des Elektrodenkabels (2) erstrecken.

2. Elektrodenvorrichtung nach Anspruch 1, **dadurch gekennzeichnet**, dass die Mittel (7) mindestens ein von der Isolierschicht (6) herausragendes flossenähnliches Teil sind.

3. Elektrodenvorrichtung nach Anspruch 1, **dadurch gekennzeichnet**, dass die Mittel (9) mindestens eine Nut in der Isolierschicht (6) sind.

4. Elektrodenvorrichtung nach Anspruch 1, **dadurch gekennzeichnet**, dass die Mittel (7, 9) teils mindestens ein von der Isolierschicht (6) herausragendes flossenähnliches Teil (7) und teils mindestens eine Nut (9) in der Isolierschicht (6) sind, wobei das Teil (7) und die Nut (9) parallel verlaufen.

5. Elektrodenvorrichtung nach einem der Ansprüche 1 bis 4, **dadurch gekennzeichnet**, dass die Steigung der wendelförmigen Mittel (7, 9) am distalen Ende (8) des Elektrodenkabels (2) geringer ist als an dem übrigen Elektrodenkabel (2).

6. Anordnung zur Durchführung einer Rotation der Elektrodenvorrichtung nach einem der Ansprüche 1 bis 5, **gekennzeichnet durch** ein Teil (10) zum Einräumen des Elektrodenkabels (2) und durch einen motorgetriebenen Rotor (11), dessen Welle (15) parallel zur Längsachse des Elektrodenkabels (2) angeordnet ist, wobei der Rotor (11) mit Mitteln (12) versehen ist, die bei einer Rotation die Mittel (7, 9) des Elektrodenkabels (2) betätigen.

7. Anordnung nach Anspruch 6, **dadurch gekennzeichnet**, dass die Mittel (12) nach aussen gerichtete Borsten sind.

## Claims

1. Electrode device for intracorporeal stimulation of body tissues, in particular for intracardiac stimulation of heart tissues, having an electrode cable, which comprises at least one elongated flexible conductor, and having an electrode head, which is attached to the distal end of the electrode cable, with the electrode cable being provided with an insulating layer, characterised in that arranged on the outside wall of the insulating layer (6) are means (7, 9) which extend in a spiral along the whole length of the electrode cable (2).

2. Electrode device according to claim 1, characterised in that the means (7) are at least one fin-like portion which protrudes from the insulating layer (6).

3. Electrode device according to claim 1, characterised in that the means (9) are at least one groove in the insulating layer (6).

4. Electrode device according to claim 1, characterised in that the means (7, 9) are partly at least one fin-like portion (7) protruding from the insulating layer (6) and partly at least one groove (9) in the insulating layer (6), with the portion (7) and the groove (9) extending in parallel.

5. Electrode device according to one of the claims 1 to 4, characterised in that the gradient of the spiral means (7, 9) at the distal end (8) of the electrode cable (2) is smaller than on the rest of the electrode cable (2).

6. Arrangement for carrying out a rotation of the electrode device according to one of the claims 1 to 5, characterised by a portion (10) for stowing away the electrode cable (2) and by a motor-driven rotor (11), the shaft (15) of which is arranged in parallel with the longitudinal axis of the electrode cable (2), with the rotor (11) being provided with means (12) which actuate the means (7, 9) of the electrode cable (2) in the event of a rotation.

7. Arrangement according to claim 6, characterised in that the means (12) are bristles which are directed towards the outside.

## Revendications

1. Dispositif d'électrode, pour la stimulation intracorporelle de tissus corporels, notamment pour la stimulation intracardiaque de tissus cardiaques, comportant un câble d'électrode, qui comporte au moins un conducteur souple oblong, et une tête d'électrode, qui est montée à l'extrémité distale du câble d'électrode, le câble d'électrode étant muni d'une couche isolante caractérisé en ce que des moyens (7,9), qui s'étendent sur toute la longueur du câble (2) d'électrode de manière hélicoïdale, sont disposés sur la paroi extérieure de la couche (6) isolante.

2. Dispositif d'électrodes suivant la revendication 1, caractérisé en ce que les moyens (7) sont sous la forme d'au moins une pièce en forme d'aileron faisant saillie de la couche (6) isolante.

3. Dispositif d'électrode suivant la revendication 1, caractérisé en ce que les moyens (7) sont sous la forme d'au moins une rainure dans la couche (6) isolante.

4. Dispositif d'électrode suivant la revendication 1 caractérisé en ce que les moyens (7,9) sont en partie sous la forme d'au moins une pièce (7) en forme d'aileron faisant saillie de la couche (6) isolante et en partie sous la forme d'au moins une rainure dans la couche (6) isolante, la pièce (7) et la rainure (9) étant parallèles.

5. Dispositif d'électrode suivant l'une des revendications 1 à 4, caractérisé en ce que le pas des moyens (7,9) en forme d'hélice est plus petit à l'extrémité (8) distale du câble (2) d'électrode que dans le reste du câble (2) d'électrode.

6. Dispositif pour réaliser une rotation du dispositif d'électrode suivant l'une des revendications 1 à 5, caractérisé par une pièce (10) pour la mise en place du câble (2) d'électrode et par un rotor (11) entraîné par un moteur, dont l'arbre (15) est disposé parallèlement à l'axe longitudinal du câble (2) d'électrode, le rotor (11) étant muni de moyens (12) qui actionnent, lors d'une rotation, les moyens (7,9) du câble (2) d'électrode.

7. Dispositif d'électrode suivant la revendication 6, caractérisé en ce que les moyens (12) sont des poils dirigés vers l'extérieur.
